# EUROPÄISCHE PATENTANMELDUNG

(11) **EP 1 262 110 A2**
(43) Veröffentlichungstag der Anmeldung: **04.12.2002**
(21) Anmeldenummer: 02008467.9
(22) Anmeldetag: 15.04.2002
(51) Int. Cl.: A23L 2/56, A23L 1/236, C07D 291/06

(54) **Nichtmetallsalz von Acesulfamsalz**

(30) Priorität: 26.04.2001 DE 10120413
(71) Anmelder: Nutrinova Nutrition Specialties & Food Ingredients GmbH, 65929 Frankfurt am Main (DE)
(72) Erfinder: Burgard, Andreas, Dr., 65929 Frankfurt (DE)
(74) Vertreter: Schweitzer, Klaus, Dr.

(57) **Zusammenfassung**

Die Erfindung betrifft eine Verbindung enthaltend ein Nichtmetallkation und ein Acesulfamanion, wobei gegebenenfalls zusätzlich Kristallwasser vorhanden sein kann. Als Nichtmetallkation enthält die Verbindung vorzugsweise ein Ammoniumkation. Die Erfindung betrifft auch ein Verfahren zur Herstellung der erfindungsgemäßen Verbindung und ihre Verwendung zum Süßen von Nahrungsmitteln und Getränken.

## Beschreibung

Der bekannte Süßstoff Acesulfam (6-Methyl-3,4-dihydro-1,2,3-oxathiazin-4-on-2,2-dioxid) wird bisher kommerziell überwiegend als Kaliumsalz (Acesulfam-K) zum Süßen von Lebensmitteln oder Zahn- und Mundpflegemitteln eingesetzt. Außerdem sind die Salze des Acesulfams mit Alkali- und Erdalkalimetallen, z.B. mit Natrium, Magnesium und Calcium bekannt. Diese Acesulfam-Metall-Komplexe zeichnen sich durch einen angenehm süßen Geschmack aus, der je nach Metallkation von einem Nachgeschmack begleitet ist. Es handelt sich um definierte Verbindungen, bei denen das Metall kationisch und das Acesulfammolekül anionisch vorliegen.

Bei bestimmten Störungen des Elektrolythaushaltes kann eine Hyperkaliämie auftreten, die besonders häufig bei Patienten mit chronischer und akuter renaler Insuffizienz, insbesondere bei Dialysepatienten, bei Diabetikern mit renaler Isuffizienz, bei Patienten mit Hypoaldosteronismus und bei Patienten mit Morbus Addison beobachtet wird. Eine Hyperkaliämie kann auch medikamentös durch kaliumsparende Diuretika wie Spironolacton, Triamteren oder Amilorid bedingt sein, die zu einer verminderten renalen Ausscheidung von Kalium führen. Hyperkaliämie kann in Extremfällen zu neuromuskulären Problemen und Arrhythmien führen, weshalb eine kaliumarme Ernährung bei Hyperkaliämie unbedingt indiziert ist.

Bisher kann jedoch ein Diabetiker mit Hyperkaliämie, die durch eine renale Insuffizienz bedingt ist, nur das Kaliumsalz des Acesulfams als Süßstoff benutzen, um die für ihn schädlichen Kohlenhydrate in der Nahrung zu substituieren.

Der Ersatz von Kalium im Acesulfam-K durch Natrium ist oft nicht erwünscht, da auf Grund einiger Herz-Kreislauferkrankungen wie z.B. Hypertonie eine natriumarme Ernährung indiziert ist. Acesulfamsalze mit Erdalkalimetallen wie z.B. Calcium oder Magnesium schmecken zwar auch süß, sie sind jedoch wegen ihres Süßprofils, bedingt durch einen mehr oder weniger metallischen Beigeschmack, keine geeignete Alternative für das Kaliumkation im Acesulfamsalz. Andere Metallkationen scheiden wegen ihrer limitierten Bekömmlichkeit von vornherein aus.

Für die vorliegende Erfindung bestand daher die Aufgabe, das Kalium-Kation im Acesulfam-salz durch ein pharmakologisch und toxikologisch unbedenkliches Kation, möglichst durch ein Nichtmetallkation, zu ersetzen, ohne dadurch die geschmacklichen und chemischen Eigenschaften, die den kommerziellen Erfolg der Verbindung Acesulfam als Süßstoff ausmachen, zu beeinträchtigen.

Gelöst wird diese Aufgabe durch eine Verbindung enthaltend ein Nichtmetallkation und ein Acesulfamanion, ggf. in Kombination mit Kristallwasser.

Als ein pharmakologisch und toxikologisch unbedenkliches Nichtmetallkation ist das Ammoniumkation bekannt. Die Federal Drug Administration (FDA) hat bisher in den USA verschiedene Ammoniumsalze wie z.B. Ammoniumbicarbonat, Ammoniumcarbonat, Ammoniumchlorid, Ammoniumhydroxid und auch das mono- und dibasische Ammoniumphosphat als Lebensmittelzusatzstoffe zugelassen (Fed. Regist. 1983, 48, 224, 52438-40). Außerdem kommt Ammonium-glycyrrhizinat seit längerem in Lebensmitteln als Süßstoff zum Einsatz (siehe M.K. Cook, Flavour Ind. 1970, 1, 12, 831-2).

Überraschenderweise wurde nun gefunden, dass das Acesulfam-ammoniumsalz genau den gleichen angenehmen Süßgeschmack besitzt wie das Acesulfam-kaliumsalz. Das Acesulfam-ammoniumsalz hat überraschenderweise exakt das gleiche Süßprofil und die gleiche Süßkraft wie das Acesulfam-kaliumsalz. Im Rahmen umfangreicher Geschmackstests konnte zwischen Acesulfam-ammonium und Acesulfam-kalium von den Prüfern kein Unterschied festgestellt werden. Überraschenderweise ist das Acesulfam-ammoniumsalz weder salzig-bitter wie z.B. das Ammoniumchloridsalz noch ist das Acesulfam-ammoniumsalz hygroskopisch wie z.B. das Ammonium-acetatsalz. Das Acesulfam-ammoniumsalz hat somit überraschend die gleichen gewünschten Eigenschaften bezüglich seiner Handhabung, seiner Süßkraft und seinem Geschmacksprofil wie das Acesulfam-kaliumsalz. Das Acesulfam-ammoniumsalz kann demnach als eine geeignete Alternative zum Acesulfam-kalium als Süßstoff bei einer kaliumarmen Ernährung bedingt durch eine renale Insuffizienz eingesetzt werden.

Zur Herstellung wird das als Ausgangssubstanz dienende Acesulfam-H, welches die dem Acesulfamanion korrespondierende Säure darstellt und nach dem in der EP-A 0 155 634 beschriebenen Verfahren hergestellt werden kann, mit Ammoniak, der in einem Lösungsmittel wie z.B. Wasser oder Alkohol gelöst vorgelegt wird, in einer Säure-Base-Reaktion zum Acesulfam-ammoniumsalz umgesetzt. Dabei reagieren Acesulfam-H als Säure und Ammoniak als Base miteinander. Wird bei der Herstellung Wasser als Lösungsmittel benutzt, so erfolgt die Isolierung des Acesulfam-ammoniumsalzes durch Kristallisation nach Verdampfen des Wassers. Wird ein Alkohol, insbesondere Methanol oder Ethanol, als Lösungsmittel eingesetzt, dann kristallisiert bei der Zugabe der Säure Acesulfam-H zur alkoholischen Ammoniaklösung, das Acesulfam-ammoniumsalz sofort aus und kann durch einfaches Filtrieren isoliert werden.

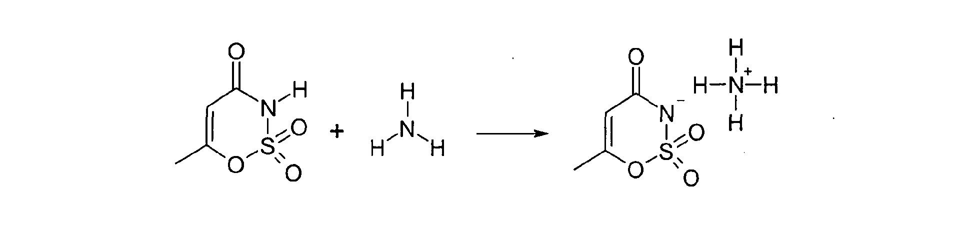

Alternativ kann auch Acesulfam-H mit Ammoniumcarbonat (NH₄)₂CO₃ beziehungsweise Ammoniumbicarbonat (NH₄)HCO₃ zu dem entsprechenden Acesulfam-ammoniumsalz umgesetzt werden, wobei Acesulfam-H als Säure und das Carbonat bzw. Bicarbonat des Ammoniumcarbonats bzw. Ammoniumbicarbonats als Base unter CO₂-Entwicklung reagieren.

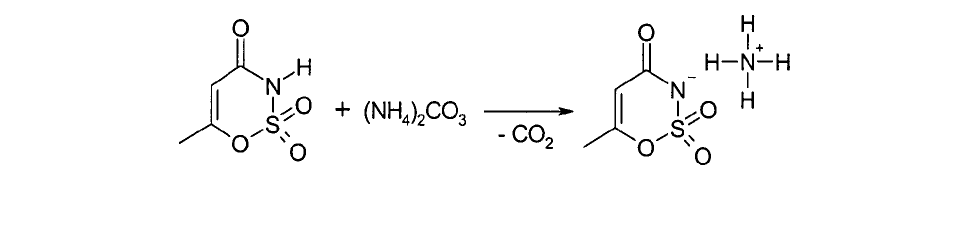

Die Kristallstruktur des Acesulfam-ammoniumsalzes konnte durch eine Röntgenstrukturanalyse aufgeklärt werden. Das Acesulfam-ammoniumsalz besteht aus orthorhombischen Kristallen, wobei das Ammoniumkation wie das Kaliumkation im Acesulfam-K (E.F. Paulus, *Acta Cryst.* 1975, **B31,** 1191) von einem negativ geladenen Stickstoffatom des Acesulfamliganden wie nachfolgend dargestellt komplexiert wird:

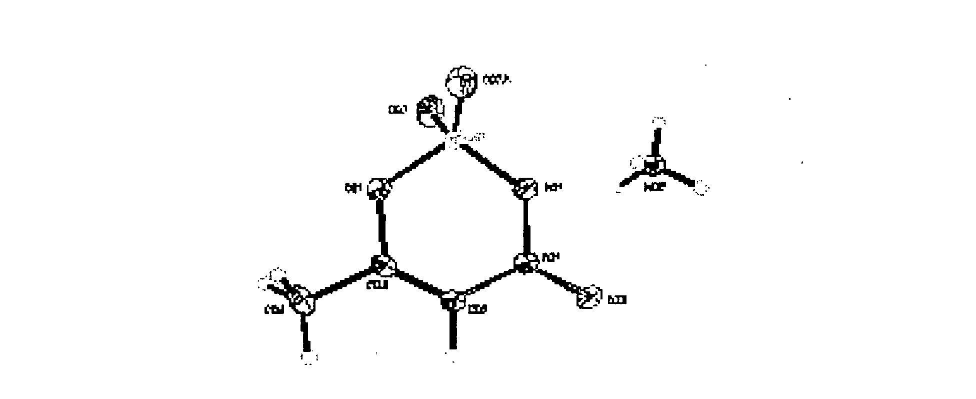

Die Erfindung soll durch die nachfolgenden Ausführungsbeispiele näher erläutert werden.

### Beispiel 1

In einem Volumen von 50 ml destilliertem Wasser wurden 100 mmol (16,3 g) Acesulfam-H gelöst, wobei sich ein pH-Wert von 0,5 einstellte. Anschließend wurde diese Lösung mit 25%-igem Ammoniakwasser versetzt, bis sich ein pH-Wert von 7,3 einstellte. Das Reaktionsgemisch wurde anschließend im Vakuum eingeengt. Es resultierten farblose, nicht hygroskopische Kristalle mit einer Ausbeute von 100 %.

### Beispiel 2

100 ml einer 20%-igen methanolischen Ammoniaklösung wurden mit 100 mmol (16,3g) Acesulfam-H versetzt. Dabei kristallisierte sofort das Acesulfam-ammoniumsalz in Form von farblosen Kristallen aus, die durch eine Filtration von der methanolischen Ammoniaklösung getrennt wurden.

Die spektroskopischen Daten des nach den Beispielen 1 und 2 hergestellten Acesulfam-ammoniumsalzes ergaben sich wie folgt:

60-MHz-¹H-NMR (d₆-DMSO): δ(ppm) = 2.1 (s, 3H, CH₃), 5.6 (s, 1H, CH), 7.5 (s, 4H, Ammonium)

## Patentansprüche

1. Verbindung enthaltend ein Nichtmetallkation und ein Acesulfamanion und gegebenenfalls zusätzlich Kristallwasser.

2. Verbindung nach Anspruch 1, **dadurch gekennzeichnet, dass** das Nichtmetallkation ein Ammoniumkation ist.

3. Verbindung nach einem oder mehreren der Ansprüche 1 bis 2, **dadurch gekennzeichnet, dass** sie ein Acesulfam-ammoniumsalz ist und dass ihre stöchiometrische Zusammensetzung Acesulfam : Ammonium im Bereich von 0,9 bis 1,1 liegt.

4. Verbindung nach einem oder mehreren der Ansprüche 1 bis 3, **dadurch gekennzeichnet, dass** sie eine ionische Verbindung ist.

5. Verfahren zur Herstellung einer Verbindung nach einem oder mehreren der Ansprüche 1 bis 4 durch Umsetzung einer in einem Lösungsmittel löslichen Acesulfam-H-Verbindung mit der basischen Vorstufe eines nichtmetallischen Kations.

6. Verfahren nach Anspruch 5, **dadurch gekennzeichnet, dass** als Vorstufe eines nichtmetallischen Kations Ammoniak oder Ammoniumcarbonat oder Ammoniumbicarbonat eingesetzt wird.

7. Verfahren nach Anspruch 5 oder 6, **dadurch gekennzeichnet, dass** als Lösungsmittel Wasser und/oder mit Wasser mischbare organische Lösungsmittel eingesetzt werden.

8. Verwendung einer Verbindung nach einem oder mehreren der Ansprüche 1 bis 4 zum Süßen von Nahrungsmitteln und Getränken.
